# FASCICULE DE BREVET EUROPEEN

(11) **EP 0 771 549 B1**
(45) Date de publication et mention de la délivrance du brevet: **26.02.2003**
(21) Numéro de dépôt: 97200128.3
(22) Date de dépôt: 07.11.1990
(51) Int. Cl.: A61F 2/01, A61M 25/00, A61M 25/04, B29C 65/02, B29C 71/02

(54) **Filtre-cathéter**
Filter-Katheter
Filter-catheter

(30) Priorité: 13.12.1989 FR 8917201
(43) Date de publication de la demande: 07.05.1997
(62) Demande divisionnaire de: 90403150.7
(73) Titulaire: Lefebvre, Jean-Marie, 59800 Lille (FR)
(72) Inventeur: Lefebvre, Jean-Marie, 59800 Lille (FR)
(74) Mandataire: 't Jong, Bastiaan Jacob

(56) Documents cités:
- FR-A- 2 606 642
- US-A- 3 713 447
- US-A- 3 799 172
- US-A- 3 938 530

## Description

La présente invention concerne un filtre-cathéter comme décrit dans le préambule de la revendication 1. Un tel cathéter est connu par le brevet FR-A-2 606 642. Les bandes flexibles entre les encochages peuvent prendre une forme boursouflée par le déplacement conduit intérieur dans la direction proximale par rapport à la canalisation. Par conséquent les bandes sont déployées à la manière de baleines d'un parapluie, jusque contre la paroi d'un vaisseau sanguin. Le filtre ainsi formé sert particulièrement à retenir les caillots sanguins.

L'invention vise à améliorer un filtre-cathéther de ce genre connu en soi.

Ce but est atteint en ce qui concerne le filtre-cathéter selon l'invention par la mesure caractéristique de la revendication 1.

Par le conduit on peut introduire de façon appropriée un produit de traitement thérapeutique à l'endroit du filtre. Un tel produit est par exemple un anti-coagulant du type héparine, ou un fibrinolitique. De ce fait les caillots sanguins captés peuvent être dissous.

D'autres caractéristiques et avantages ressortiront plus clairement de la description qui va être faite du mode préféré de réalisation du filtre-cathéter à usage temporaire, illustré par le dessin annexé dans lequel:
- La figure 1: est une vue schématique en coupe du filtre au repos;
- La figure 2: est une vue schématique en coupe du filtre déployé;
- La figure 3: est une vue en coupe transversale du filtre selon l'axe D de la figure 2;
- La figure 4: est une vue schématique en coupe de l'extrémité distale d'un filtre;
- La figure 5: est une vue schématique de côté du dispositif de maintien du filtre-cathéter pendant le traitement de thermofixation.

Le filtre-cathéter 1 de l'invention se compose essentiellement d'une canalisation 2 creuse et d'un conduit creux 3 qui est placé coaxialement à l'intérieur de la canalisation 2. Ces deux éléments, canalisation 2 et conduit 3 sont en polytétrafluoréthylène.

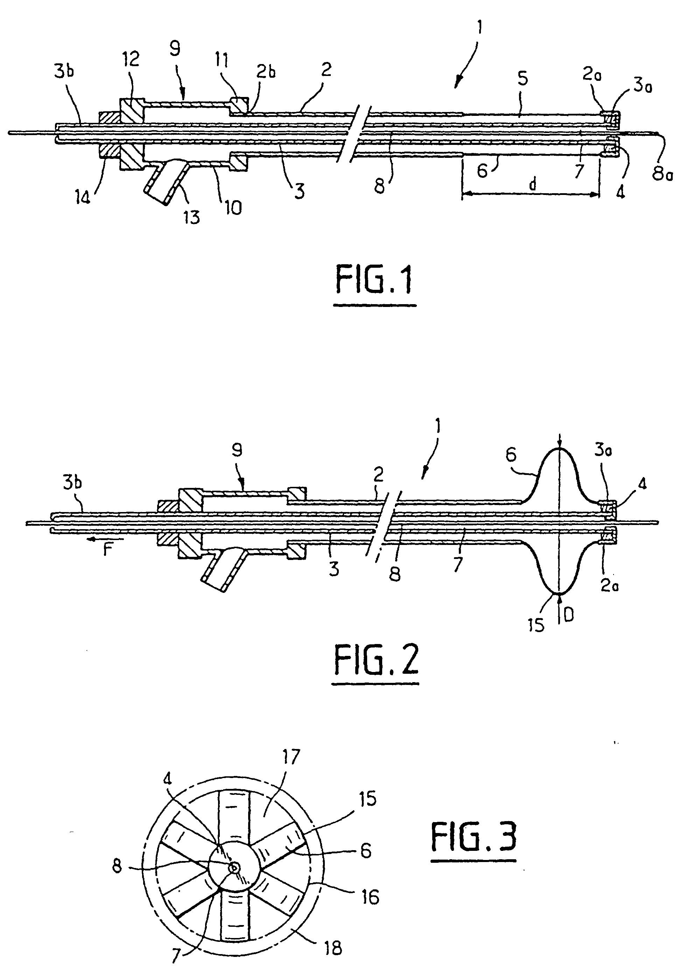

Dans le présent texte les termes "proximal" et "distal" sont employés en prenant pour référence la partie du corps du patient par où le filtre est introduit. Ainsi l'extrémité distale du filtre, représentée à droite sur les figures 1 et 2, est l'extrémité la plus éloignée de cette partie du corps, et l'extrémité proximale la plus rapprochée.

Les extrémités distales respectivement 2a de la canalisation 2 et 3a du conduit 3 sont thermosoudées ensembles et autour d'un anneau métallique 4, en acier inoxydable, platine ou or. Cet anneau 4 est dans un matériau radio-opaque, détectable lors du positionnement du filtre.

Comme cela ressort clairement des figures 1 et 2, l'anneau 4 est placé entre la canalisation 2 et l'extrémité distale 3a du conduit 3, l'extrémité distale 2a de la canalisation étant repliée à l'intérieur du conduit 3 et recouvrant l'extrémité distale 3a dudit conduit. Le thermosoudage réalise l'assemblage de ces éléments pour former l'extrémité 15 du filtre-cathéter.

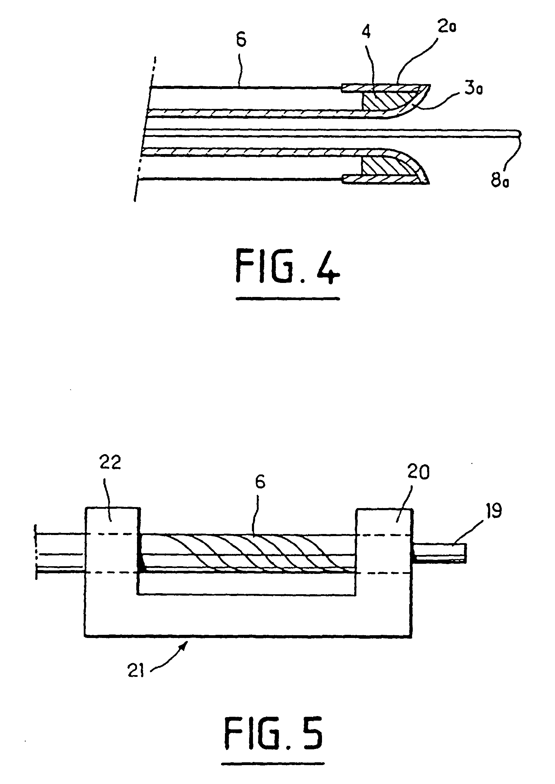

Selon un autre mode de réalisation illustré à la figure 4, le thermosoudage intervient alors que l'extrémité distale 3a du conduit intérieur 3 déborde au-delà de l'anneau métallique 4 et s'évase vers l'extérieur venant au contact de l'extrémité distale 2a de la canalisation 2.

En amont de son extrémité distale 2a, la canalisation 2 comporte six entailles 5, pratiquées dans le sens longitudinal sur une longueur d de 30 mm; elles sont régulièrement réparties sur la périphérie de la canalisation. Ces entailles 5 délimitent six bandes 6 identiques, chacune d'elles occupe un espace angulaire de 60° de la canalisation 2. Pour une canalisation 2 de 3mm de diamètre, chaque bande 6 a une largeur d'environ 1,5mm.

Le conduit 3 est creux et comporte un canal central 7, dans lequel peut librement coulisser une tige métallique 8 servant de tige de guidage lors de l'introduction du filtre-cathéter.

Vers l'extrémité proximale, mais à l'extérieur du corps du patient, la canalisation 2 est fixée sur un dispositif 9 de maintain et d'alimentation. Ce dispositif 9 comporte une tubulure cylindrique et des baques 11 et 12 d'extrémité. La première baque 11 annulaire raccorde l'extrémité proximale 2b de la canalisation 2 à la partie avant de la tubulure 10. La seconde baque 12 entoure le conduit 3 et ferme la partie arrière de la tubulure 10. Un coude 13 débouche en oblique dans le corps de la tubure 10.

L'extrémité proximale 3b du conduit 3 va au-delà de la tubulure 10. La baque 12 enserre cette extrémité distale 3b de façon étanche; toutefois le conduit 3 peut coulisser longitudinalement dans la bague 12 par rapport à la tubulure 10 et donc à la canalisation 2.

Une baque de blocage 14 est apte à assurer le blocage du conduit 3 contre la bague 12.

La tubulure 10 est fixée de manière connue à proximité du patient sur un bâti supportant deux systèmes d'alimentation de produits à injecter, débouchant pour le premier dans l'extrémité proximale 3b du conduit et pour le second dans le coude 13 de la tubulure 10.

La mise en oeuvre du filtre-cathéter se déroule dans les conditions suivantes.

Dans une première phase, la tige de guidage 8 est introduite par voie percutanée dans la veine adéquate, jusqu'à ce que son extrémité 8a distale se trouve en position dans la veine cave inférieure du patient.

Dans une deuxième phase, on fait coulisser depuis l'extérieur du corps du patient le filtre-cathéter 1 le long de la tige de guidage 8 jusqu'à ce que l'anneau 4 se trouve en position dans la veine cave, là où le filtre doit être implanté.

Pendant le déroulement de cette deuxième phase, les bandes 6 sont au repos, c'est-à-dire qu'elles sont dont le prolongement de la canalisation 2.

Dans une troisième phase illustrée par la figure 2 et correspondant au déploiement du filtre, la baque de blocage 14 est desserrée en sorte de permettre au conduit 3 de coulisser dans la bague 12. Puis on déplace avec précaution l'extrémité proximale 3b du conduit 3 vers l'arrière dans le sens de la flèche F. Dans cette phase, tout le conduit 3 se déplace ainsi que l'anneau 4, solidaire de l'extrémité distale 3a du conduit. Etant donné que la tubulure 10 et donc la canalisation 2 sont maintenues en position, le recul de l'anneau 4 dans le sens de la flèche F ne peut se produire que grâce à la présence des bandes 6 flexibles. Chaque bande 6 se plie individuellement, créant de ce fait une deformation transversale homogène de la canalisation dans la zone des entailles 5. Le déplacement et la longueur des bandes 6 sont choisis de telle sorte que, en position de déploiement, les parties médianes 15 des bandes 6, correspondant au plan D de courbure, prennent appui sur la paroi interne 16 de la veine. Une fois que le filtre est correctement déployé, la baque de blocage 14 est resserrée en sorte d'empêcher tout déplacement du conduit 3 par rapport à la canalisation 2 et donc de maintenir les bandes 6 dans leur état de déformation transversale.

Comme cela apparaît clairement sur la figure 3, qui est une coupe selon le plan D, les six bandes 6 sont déployées transversalement par rapport à la canalisation 2 et obturent partiellement l'intérieur 17 de la veine 18. Ainsi les caillots sanguins se déplaçant à l'intérieur 17 de la veine 18 sont arrêtés par les bandes 6 tandis que le sang peut circuler librement.

Préalablement à l'utilisation du filtre-cathéter, on a fait subir à celui-ci un traitement thermique de thermofixation. Ce traitement a consisté à donner un choc thermique à la partie distale du filtre alors que les bandes flexibles 6 sont dans leur état déployé, comme illustré à la figure 2. La durée de ce traitement et la température sont déterminées en sorte que la forme prise par les bandes soit en quelque sorte mémorisée dans la structure polymérique du polytétrafluoréthylène. Ce traitement augmente la résistance à la déformation des bandes 6 et évite l'affaissement du filtre à l'intérieur de la veine 18 et le risque de thrombogénèse dû aux turbulences créées dans le flux sanguin par l'hétérogénéité de disposition des bandes 6 dans la veine.

Selon un autre mode de réalisation du traitement de thermofixation illustré à la figure 5, le traitement thermique est appliqué sur la partie distale du filtre-cathéter alors que les bandes flexibles 6 ont une forme torsadée. Pour cela, on emmanche dans le conduit intérieur 3 un mandrin 19 de manière à rigidifier la partie distale du filtre-cathéter; on bloque en position l'extrémité distale 2a de la canalisation 2 dans la branche avant 20 d'un support de maintain 21, on fait subir à la canalisation 2 une rotation sur elle-même, qui confère aux bandes flexibles 6 la forme torsadée illustrée sur la figure 5, puis on bloque en position la canalisation 2 en amont desdites bandes flexibles 6 dans la branche arrière 22 du supports de maintain 21. Le support 21 et la partie distale du filtre-cathéter sont placés dans un four de cuisson par exemple par rayonnement micro-onde pour y subir le traitement thermique, pendant 4 à 5 minutes. On remarque que lors de l'utilisation du filtre, les bandes flexibles 6 ont tendance à prendre la forme en hélice conférée lors du traitement thermique. Bien sûr sur le support de maintain 21, les bandes 6 peuvent être dans la position repos comme représenté sur la figure 5 ou dans l'état déployé.

Un produit de traitement thérapeutique peut être injecté en amont du filtre à travers le conduit 3, à partir de l'extrémité proximale 3b de celui-ci. Il s'agit d'un anticoagulant du type héparine ou d'un fibrinilotique. Le conduit 3 peut comporter des orifices, par exemple disposés en spirale, dans la zone faisant face aux bandes 6. Ainsi le produit de traitement thérapeutique introduit dans le conduit 3 peut être réparti à la fois en amont et au niveau du filtre lui-même.

Le même produit, ou éventuellement un autre produit de traitement thérapeutique peut être injecté dans la zone immédiatement en aval du filtre à travers la canalisation 2, à partir du coude 13 de la tubulure 10.

Une fois le traitement jugé satisfaisant, le retrait du filtre s'effectue de la manière suivante. La baque de blocage 14 est desserrée et l'extrémité proximale 3b du conduit 3 est repoussée dans le sens inverse de la flèche F jusqu'à ce que les languettes 6 retrouvent leur position de repos, rectilignes et parallèles entre elles. Puis, la baque 14 est de nouveau resserrée, et l'ensemble conduit 3 et canalisation 2 est retiré de la veine.

Les essais réalisés à l'aide du filtre-cathéter selon l'invention ont permis de constater l'absence de toute formation de caillots sanguins du fait de la présence du filtre dans la veine.

La présente invention n'est pas limitée au mode de réalisation qui a été décrit à titre d'exemple non exhaustif, mais en couvre toutes les variantes qui découlent de la revendication 1. En particulier la tige de guidage 8 peut être creuse et permettre l'injection de produit de traitement en aval du filtre voire même bien au-delà de l'extrémité distale du filtre-cathéter 1.

## Revendications

1. Filtre-cathéter est distiné à interrompre partiellement et au moins temporairement un vaisseau sanguin, comprenant une canalisation (2) avec une extrémité distale et une extrémité proximale, où sont mises à son extrémité distale, symétriquement réparties sur la périphérie, des entailles longitudinales délimitant des bandes flexibles (6), se trouvant dans une lumière de la canalisation (2) le conduit intérieur (3) avec une extrémité distale et une extrémité proximale, où la canalisation (2) et le conduit intérieur (3) sont solidaires à leurs extrémités distales (2a, 3a) **caractérisé en ce qu'**entre la lumière de la canalisation (2) et le conduit intérieur (3) s'est formé un conduit fait pour introduire un produit de traitement qui est en communication d'une part avec l'élément filtrant et d'autre part avec un espace intérieur d'un dispositif (9) de maintien et d'alimentation essentiellement cylindrique et coaxial avec la canalisation (2), fixé par une de ses extrémités à cette canalisation (2) et enserrant par son autre extrémité le conduit intérieur (3) en permettant le coulissement de celui-ci de façon étanche.

2. Filtre-cathéter selon la revendication 1, **caractérisé en ce que** le dispositif (9) de maintien et d'alimentation comprennent des moyens d'étanchéité pour l'application coulissante du conduit intérieur.

## Patentansprüche

1. Filter-Katheter zur teilweisen und zumindest zeitweiligen Unterbrechung eines Blutgefäßes, mit einem Kanal (2) und mit einem distalen Ende und einem proximalen Ende, wobei an seinem distalen Ende symmetrisch über den Umfang verteilt Längseinschnitte vorgesehen sind, die flexible Bänder (6) begrenzen, wobei sich in einem Lumen des Kanals (2) eine innere Leitung (3) mit einem distalen Ende und einem proximalen Ende befindet, wobei der Kanal (2) und der inneren Leitung (3) an ihren distalen Enden (2a, 3a) fest miteinander verbunden sind, **dadurch gekennzeichnet, daß** zwischen dem Lumen des Kanals (2) und der inneren Leitung (3) eine Leitung gebildet ist für das Einführen eines Behandlungsmittels, wobei die Leitung einerseits mit dem Filterelement und andererseits mit einem Innenraum einer Einrichtung (9) zum Halten und zum Versorgen verbunden ist, die im wesentlichen zylindrisch und koaxial zum Kanal (2) ist, an einem ihrer Enden mit diesem Kanal (2) fest verbunden ist und an einem ihrer anderen Ende die innere Leitung (3) einsperrt, jedoch eine Verschiebbarkeit in abdichtende Weise erlaubt.

2. Filter-Katheter nach Anspruch 1 **dadurch gekennzeichnet, daß** die Halte- und Versorgungseinrichtung (9) Abdichtmittel zur gleitenden Aufnahme der inneren Leitung aufweist.

## Claims

1. Filter-catheter is intended to partially and at least temporarily interrupt a blood vessel, comprising a pipe (2) with a distal end and a proximal end, in which are placed at its distal end, symmetrically distributed around its periphery, longitudinal slots defining flexible strips (6), situated in a hole in the pipe (2) the internal conduit (3) with a distal end and a proximal end, in which the pipe (2) and internal conduit (3) are firmly attached at their distal ends (2a, 3a), **characterised by** the fact that between the hole in the pipe (2) and the internal conduit (3) is formed a conduit created for introduction of a treatment product which is in communication on the one hand with the filtering element and on the other with an internal space of a holding and supply device (9) which is essentially cylindrical and coaxial with the pipe (2), fixed by one of its ends to this pipe (2) and with its other end enclosing the internal conduit (3) while permitting sliding of this in sealed manner.

2. Filter-catheter as described in claim 1, **characterised by** the fact that the holding and supply device (9) includes sealing means for sliding application of the internal conduit.
